# EUROPEAN PATENT SPECIFICATION

(11) **EP 4 088 689 B1**
(45) Date of publication and mention of the grant of the patent: **05.06.2024**
(21) Application number: 22173362.9
(22) Date of filing: 13.05.2022
(51) Int. Cl.: A61F 2/24

(54) **SYSTEMS AND KIT FOR SEPARATING NATIVE HEART VALVE LEAFLETS ATTACHED TOGETHER BY A FIXATION DEVICE**
SYSTEME UND KIT ZUR TRENNUNG VON DURCH EINE FIXIERVORRICHTUNG MITEINANDER VERBUNDENEN NATIVEN HERZKLAPPENFLÜGELN
SYSTÈMES ET KIT DE SÉPARATION DE FEUILLETS VALVULAIRES CARDIAQUES NATIFS ATTACHÉS ENSEMBLE PAR UN DISPOSITIF DE FIXATION

(30) Priority: 14.05.2021 US 202163188614 P
(43) Date of publication of application: 16.11.2022
(73) Proprietor: Evalve, Inc., Santa Clara, CA 95054 (US)
(72) Inventor: OSTERBAUER, Philip, Wyoming, 55092 (US); EIDENSCHINK, Tracee, Wayzata, 55391 (US); DALE, Ted, Corcoran, 55340 (US); CHILDS, Richard, San Francisco, 94115 (US); WEI, Michael F, Redwood City, 94065 (US)
(74) Representative: Boult Wade Tennant LLP

(56) References cited:
- WO-A1-2021/007324
- US-A1- 2020 121 460
- US-A1- 2020 323 528

## Description

### RELATED APPLICATIONS

This application claims priority to U.S. Provisional Application No. 63/188,614 filed May 14, 2021.

### BACKGROUND

Mitral valve regurgitation is characterized by retrograde flow from the left ventricle of a heart through an incompetent mitral valve into the left atrium. During a normal cycle of heart contraction (systole), the mitral valve acts as a check valve to prevent flow of oxygenated blood back into the left atrium. In this way, the oxygenated blood is pumped into the aorta through the aortic valve. Regurgitation of the valve can significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles themselves or the left ventricular wall may be damaged or otherwise dysfunctional. Commonly, the valve annulus can be damaged, dilated, or weakened, limiting the ability of the mitral valve to close adequately against the high pressures of the left ventricle.

The most common treatments for mitral valve regurgitation rely on valve replacement or repair including leaflet and annulus remodeling, the latter generally referred to as valve annuloplasty. One technique for mitral valve repair which relies on suturing adjacent segments of the opposed valve leaflets together is referred to as the "bow-tie" or "edge-to-edge" technique. While all these techniques can be effective, they often rely on open heart surgery where the patient's chest is opened, frequently via a sternotomy, with the patient placed on cardiopulmonary bypass. The need to both open the chest and place the patient on bypass can be traumatic.

In some patients, a fixation device can be installed into the heart using minimally invasive techniques. The fixation device can hold the adjacent segments of the opposed valve leaflets together and may reduce mitral valve regurgitation. One such device used to clip the anterior and posterior leaflets of the mitral valve together is the MitraClip^{®} fixation device, sold by Abbott Vascular, Santa Clara, Calif., USA.

Sometimes after a fixation device is installed mitral valve regurgitation can still exist, or can arise again. Further, other problems with the heart may arise that can make it desirable for the fixation device to be disabled or removed. For example, it can be desirable to remove the fixation device to allow for implantation of a replacement heart valve. For at least these reasons, it is desirable to provide systems and methods for removing or disabling fixation devices that are already installed.

In WO 2021/007324 A1, a retrieval catheter and methods of use are described for removing a heart valve therapy such as a leaflet clip or artificial leaflet cord. The retrieval catheter can include a cutting element and a basket, piercing element, clamping mechanism, or similar grasping device. The method includes delivering a catheter to the region of the heart valve therapy and then manipulating the catheter and associated instruments to cut tissue as necessary and then remove the heart valve therapy and withdraw the catheter.

### SUMMARY

The purpose and advantages of the disclosed subject matter will be set forth in and apparent from the description that follows, as well as will be learned by practice of the disclosed subject matter. For purpose of illustration and not limitation, the various embodiments described herein relate to systems and methods for separating native heart valve leaflets attached together by a fixation device. Additional advantages of the disclosed subject matter will be realized and attained by the systems and methods particularly pointed out in the written description and claims hereof, as well as from the appended drawings.

To achieve these and other advantages, and in accordance with the purpose of the disclosed subject matter, as embodied and broadly described, the disclosed subject matter includes systems for separating native heart valve leaflets attached together by a fixation device. Systems in accordance with the disclosed subject matter include an elongate shaft including a proximal end portion, a distal end portion and a longitudinal axis extending therebetween. The elongate shaft is configured for transvascular delivery of the distal end portion to a native heart valve. A balloon is disposed at the distal end portion of the elongate shaft, and the balloon is inflatable from a collapsed condition to an inflated condition. At least one snare lumen extends along at least a portion of the balloon, the at least one snare lumen having an exit port defined therein, wherein the exit port is disposed at an intermediate location along a length of the balloon. A snare is deployable through the exit port from a delivery position within the at least one snare lumen to an extended position extending beyond the exit port. The snare in the extended position is configured to capture the fixation device.

For purpose of example, the elongate shaft can include a braided reinforcement. Additionally or alternatively, the elongate shaft can include a hypotube. Additionally or alternatively, the elongate shaft can include at least one of stainless steel, nitinol, mp35, polyimide, pebax, and braid reinforced polyimide. Additionally or alternatively, the elongate shaft can be configured to transmit torque along the longitudinal axis and the exit port of the at least one snare lumen can be moved circumferentially about the longitudinal axis by rotation of the proximal end portion.

For purpose of example and not limitation, the balloon can be rotatable about the longitudinal axis of the elongate shaft to move the exit port of the at least one snare lumen circumferentially about the longitudinal axis. Additionally or alternatively, the balloon can be configured to unfold upon inflation from the collapsed condition to the inflated condition.

For purpose of example and not limitation, the at least one snare lumen can extend along an exterior of the balloon and can be bonded to a portion of the balloon. Additionally or alternatively, a segment of the at least one snare lumen can be attached to at least one of the elongate shaft and the balloon, and the at least one snare lumen can extend along an exterior of the balloon and can be movable relative the exterior of the balloon as the balloon inflates from the collapsed condition to the inflated condition to accommodate an increased circumference of the balloon. Additionally or alternatively, the at least one snare lumen can be defined within a wall of the balloon. Additionally or alternatively, the at least one snare lumen can be configured to increase in length axially as the balloon inflates from the collapsed condition to the inflated condition to accommodate an increased circumference of the balloon. Additionally or alternatively, the at least one snare lumen can include between one and four snare lumens spaced about an outer circumference of the balloon.

For example and not limitation, least one of the balloon and the at least one snare lumen can include a radiopaque marker proximate the exit port.

For example and not limitation, a guidewire lumen can extend along at least a length of the elongate shaft.

For example and not limitation, the balloon can be concentric with the longitudinal axis of the elongate shaft. Additionally or alternatively, the balloon can be acentric with the longitudinal axis of the elongate shaft.

For example and not limitation, the balloon can have an inflation diameter in the inflated condition and the inflation diameter can be larger than a maximum cross dimension of an orifice defined between the native heart valve leaflets to be separated.

For example and not limitation, at least one cutter can be disposed along at least a portion of the balloon. For example and not limitation, the at least one cutter can be disposed between about 30 degrees and 180 degrees about an outer circumference of the balloon from the at least one snare lumen.

The disclosed subject matter further includes methods for separating native heart valve leaflets attached together by a fixation device. Methods in accordance with the disclosed subject matter include delivering a distal end portion of a system for separating native heart valve leaflets attached together by a fixation device as described above to a native heart valve. Methods in accordance with the disclosed subject matter further include deploying a snare from a delivery position within the at least one snare lumen to an extended position extending beyond the exit port. Methods in accordance with the disclosed subject matter further include capturing the fixation device within the snare with the snare in the extended position, and inflating the balloon from a collapsed condition to an inflated condition to separate the native heart valve leaflets.

For example and not limitation, delivering the distal end portion can include positioning the balloon through an orifice defined between the native heart valve leaflets. For example and not limitation, the distal end can be delivered transapically. Additionally or alternatively, the distal end can be delivered transeptally.

For example and not limitation, deploying the snare can include rotating the balloon about the longitudinal axis of the elongate shaft to align the exit port towards the fixation device.

For example and not limitation, the native heart valve can be a mitral valve. Additionally or alternatively, delivering the distal end portion can include positioning the balloon through a lateral orifice defined between the native mitral valve leaflets. Additionally or alternatively, methods can include rotating the balloon about the longitudinal axis of the elongate shaft to align the exit port away from the native mitral valve anterior leaflet after capturing the fixation device. Additionally or alternatively, inflating the balloon can tear the native mitral valve anterior leaflet. Additionally or alternatively, the fixation device can remain attached to the native mitral valve posterior leaflet.

Additionally or alternatively, methods can include weakening native mitral valve anterior leaflet tissue before inflating the balloon. For example and not limitation, at least one of a needle, blade, RF energy, and hydrogen peroxide can be applied to the native mitral valve anterior leaflet tissue in order to weaken it.

Additionally or alternatively, the balloon can include a cutter disposed along at least a portion thereof, and methods can include aligning the least one cutter with the native mitral valve anterior leaflet.

Additionally or alternatively, methods can include positioning a second balloon through a second orifice on an opposing side of the fixation device, and inflating the second balloon.

The disclosed subject matter further includes kits for treating a native heart valve having leaflets attached together by a fixation device. Kits in accordance with the disclosed subject matter include a system for separating native heart valve leaflets attached together by a fixation device having any of the features described above. Kits in accordance with the disclosed subject matter also include a prosthetic heart valve.

It is to be understood that both the foregoing general description and the following detailed description are exemplary and are intended to provide further explanation of the disclosed subject matter claimed.

The accompanying drawings, which are incorporated in and constitute part of this specification, are included to illustrate and provide a further understanding of the systems and methods of the disclosed subject matter. Together with the description, the drawings serve to explain the principles of the disclosed subject matter.

### BRIEF DESCRIPTION OF THE DRAWINGS

FIG. 1 illustrates the left ventricle and left atrium of the heart during systole.
FIG. 2. illustrates an exemplary fixation device clipping together the anterior and posterior leaflets of a mitral valve.
FIG. 3A illustrates a cross-sectional side view of a mitral valve having a fixation device clipping together the anterior and posterior leaflets of the mitral valve.
FIG. 3B illustrates a cross-sectional side view of the mitral valve of FIG. 3A after separation of the anterior and posterior leaflets with the fixation device remaining clipped to the posterior leaflet.
FIG. 4A is a side view of an exemplary system for separating native heart valve leaflets in accordance with the disclosed subject matter with the balloon in the collapsed condition.
FIG. 4B is a side view of the exemplary system of FIG. 4A with the balloon in the inflated condition.
FIG. 4C is a cross-sectional view of the exemplary system of FIG. 4A with the balloon in the inflated condition taken along line 4C-4C, as depicted in FIG. 4B.
FIG. 5A is a side view of another exemplary system for separating native heart valve leaflets in accordance with the disclosed subject matter with the balloon in the inflated condition.
FIG. 5B is a cross-sectional view of the exemplary system of FIG. 5A taken along line 5B-5B, as depicted in FIG. 5A.
FIG. 6 is a side view of another exemplary system for separating native heart valve leaflets in accordance with the disclosed subject matter with the balloon in the inflated condition.
FIG. 7A is a cross-sectional view of a native mitral valve taken along the native mitral valve annulus looking down from the left atrium. The native mitral valve has fixation devices clipping together the native heart valve leaflets, and an exemplary system for separating native heart valve leaflets in accordance with the disclosed subject matter is positioned within the native mitral valve with the balloon in the collapsed condition.
FIG. 7B is a front cross-sectional view of the heart valve and exemplary system of FIG. 7A taken along line 7B-7B, as depicted in FIG. 7A.
FIG. 8A is the cross-sectional view of the native mitral valve and exemplary system of FIG. 7A with the snare deployed to capture the fixation devices.
FIG. 8B is the cross-sectional view of the native mitral valve and exemplary system of FIG. 7A with the balloon inflating from the collapsed condition to the inflated condition and the exit port of the at least one snare lumen moving circumferentially about the longitudinal axis of the elongate shaft.
FIG. 8C is the cross-sectional view of the native mitral valve and exemplary system of FIG. 7A with the balloon in the inflated condition and the leaflets of the native mitral valve separated.
FIG. 9A is a front cross-sectional view of a native mitral valve and a transaortically delivered device for weakening tissue of the native mitral valve anterior leaflet tissue.
FIG. 9B is a front cross-sectional view of a native mitral valve and a transeptally delivered device for weakening tissue of the native mitral valve anterior leaflet tissue.
FIG. 10 is the cross-sectional view of the native mitral valve and exemplary system of FIG. 7A with a second balloon positioned within the native mitral valve.
FIG. 11 is a side view of another exemplary system for separating native heart valve leaflets in accordance with the disclosed subject matter with the balloon in the inflated condition.

### DETAILED DESCRIPTION

Reference will now be made in detail to the various exemplary embodiments of the disclosed subject matter, which are illustrated in the accompanying drawings. The structure and corresponding method of operation of the disclosed subject matter will be described in conjunction with the detailed description of the system. The accompanying drawings, where like reference numerals refer to identical or functionally similar elements throughout the separate views, serve to further illustrate various embodiments and to explain various principles and advantages all in accordance with the disclosed subject matter.

The disclosed subject matter is directed to systems and methods for separating native heart valve leaflets attached together by a fixation device. Although embodiments described herein are directed to separating native mitral valve leaflets attached together by a fixation device, it will be understood that systems and methods in accordance with the disclosed subject matter can also be applied to separate leaflets of other heart valves attached together by a fixation device, such as the tricuspid valve, aortic valve, or pulmonary valve. Additionally, the term fixation device as used herein encompasses both the singular-fixation device-as well as a plurality of fixation devices. That is, it is understood that one or more fixation devices may be used to attach native heart valve leaflets together, and systems and methods in accordance with the disclosed subject matter can be used to separate the native heart valve leaflets attached together by the one or more fixation devices.

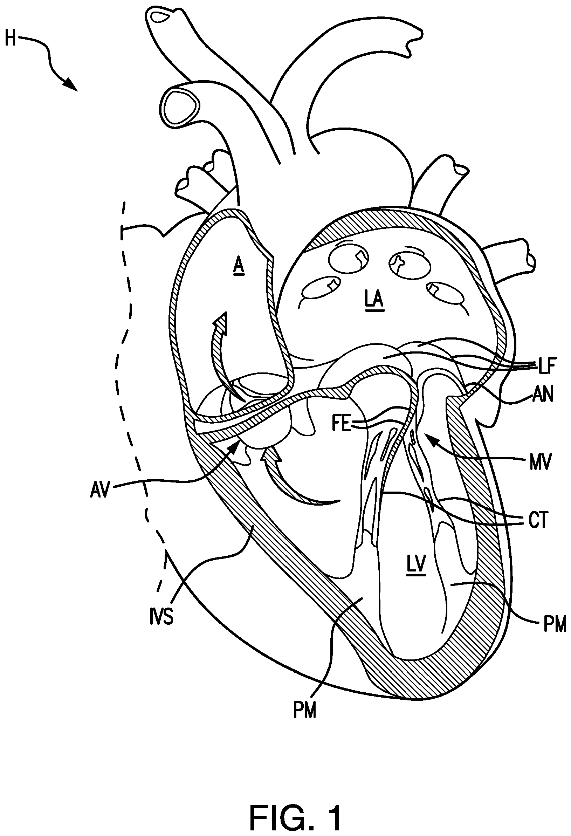

The left ventricle (LV) of a normal heart H in systole is illustrated in FIG. 1. The left ventricle (LV) is contracting and blood flows outwardly through the tricuspid (aortic) valve (AV) in the direction of the arrows. Back flow of blood or "regurgitation" through the mitral valve (MV) is prevented since the mitral valve is configured as a "check valve" which prevents back flow when pressure in the left ventricle is higher than that in the left atrium (LA). The mitral valve (MV) comprises a pair of leaflets having free edges (FE) which meet evenly to close, as illustrated in FIG. 1. The opposite ends of the leaflets (LF) are attached to the surrounding heart structure along an annular region referred to as the annulus (AN). The free edges (FE) of the leaflets (LF) are secured to the lower portions of the left ventricle LV through chordae tendinae (CT) which include a plurality of branching tendons secured over the lower surfaces of each of the valve leaflets (LF). The chordae (CT) in turn, are attached to the papillary muscles (PM) which extend upwardly from the lower portions of the left ventricle and intraventricular septum IVS.

A number of structural defects in the heart can cause mitral valve regurgitation. Regurgitation occurs when the valve leaflets do not close properly allowing leakage from the ventricle into the atrium. For example, enlargement of the heart can cause the mitral annulus to become enlarged such that the free edges (FE) of the mitral valve do not meet during systole. This can result in a gap which allows blood to leak through the valve during ventricular systole. Ruptured or elongated chordae tendinae can also cause one or more valve leaflets to prolapse such that the two valve leaflets do not properly meet and leakage occurs from the left ventricle into the left atrium. Such regurgitation can also occur in patients who have suffered ischemic heart disease where the left ventricle does not contract sufficiently to effect proper closure.

Fixation devices can be used for grasping, approximating and fixating tissues such as valve leaflets to treat cardiac valve regurgitation, particularly mitral valve regurgitation. Additional examples and details related to fixation devices, delivery devices for directing fixation devices to a targeted treatment area, handles, and deployment mechanisms, are described in U.S. Patent Number 7,666,204, U.S. Patent Number 7,563,267, and U.S. Patent Application Publication No. 2017/0100250. One such device used to clip the anterior and posterior leaflets of the mitral valve together is the MitraClip^{®} fixation device, sold by Abbott Vascular, Santa Clara, Calif., USA.

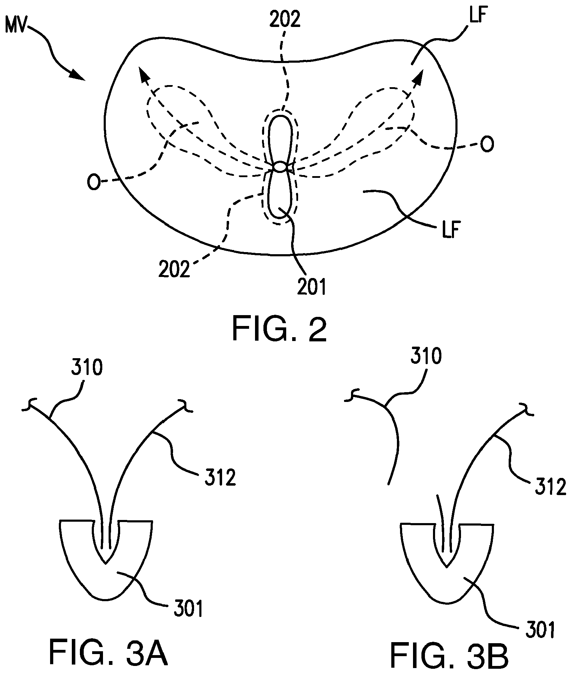

FIG. 2 illustrates an exemplary fixation device clipping together the anterior and posterior leaflets of a mitral valve. FIG. 2 is a taken from the atrial side of the mitral valve (MV), and therefore, the atrial elements 201 of the fixation device are shown in solid line and the ventricular elements 202 are shown in dashed line. The leaflets LF are held in place so that during diastole, as shown in FIG. 2, the leaflets LF remain in position between the atrial elements 201 and ventricular elements 202 surrounded by openings or orifices O which result from the diastolic pressure gradient. As depicted in FIG. 2, the leaflets LF can be coapted or attached together such that their upstream surfaces are facing each other in a vertical orientation, parallel to the direction of blood flow through mitral valve MV.

FIG. 3A illustrates a side cross-sectional view of a mitral valve having native leaflets attached together by a fixation device 301. As embodied herein, the fixation device 301 can be coupled to the anterior leaflet 310 and posterior leaflet 312. As described further herein, separating the native leaflets may be desirable to facilitate further treatment of the native mitral valve, such as by installing a replacement heart valve, or to satisfy another clinical need. However, fixation devices can be embedded with the leaflet tissue and/or other surrounding tissues as a result of tissue ingrowth, making it difficult to extract the implant and/or separate the native leaflets. With reference to FIG. 3B, separation of the native leaflets can be achieved by tearing, cutting, or severing the tissue of one or more of the leaflets. For example, and as embodied herein, separation can be achieved by tearing the native mitral valve anterior leaflet tissue with the fixation device 301 remaining attached to the posterior leaflet 312 after the leaflets are separated. As described further herein, tearing of the anterior leaflet tissue can be advantageous, as there is less risk that the fixation device will interfere with functioning of the left ventricular outflow tract when the fixation device remains attached to the posterior leaflet.

Systems for separating native heart valve leaflets attached together by a fixation device in accordance with the disclosed subject matter generally include an elongate shaft having a proximal end portion, a distal end portion and a longitudinal axis extending therebetween. The elongate shaft is configured for transvascular delivery of the distal end portion to a native heart valve. A balloon is disposed at the distal end portion of the elongate shaft, and the balloon is inflatable from a collapsed condition to an inflated condition. At least one snare lumen extends along at least a portion of the balloon, the at least one snare lumen having an exit port defined therein. A snare is deployable through the exit port from a delivery position within the at least one snare lumen to an extended position extending beyond the exit port. The snare in the extended position is configured to capture the fixation device.

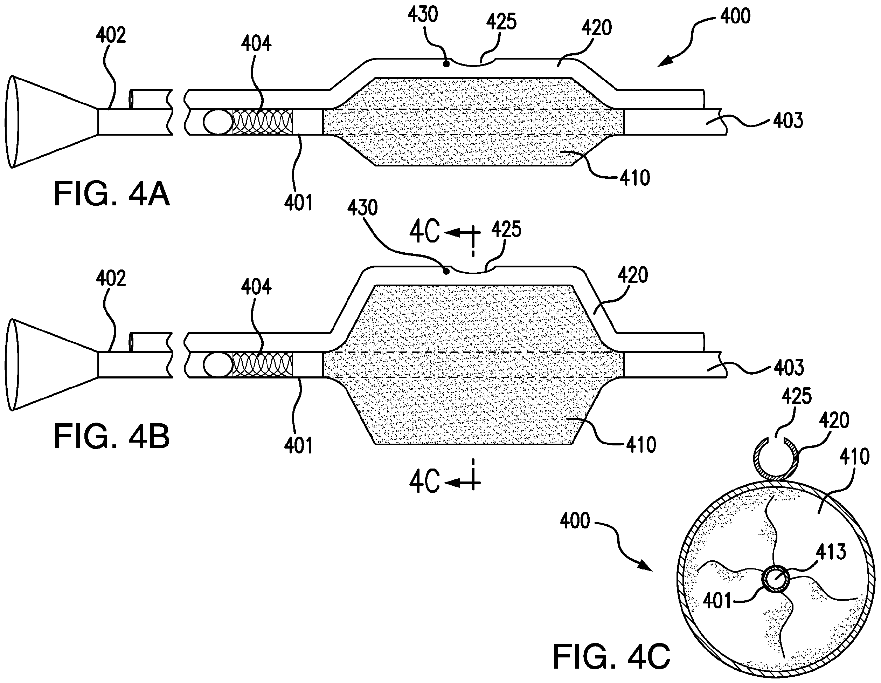

For purpose of illustration and not limitation, reference is made to the exemplary embodiment of a system 400 for separating native heart valve leaflets attached together by a fixation device shown in FIGs. 4A-4C. The system 400 generally includes an elongate shaft 401 having a proximal end portion 402, a distal end portion 403, and a longitudinal axis extending therebetween. The elongate shaft 401 is configured for transvascular delivery of the distal end portion to a native heart valve. Techniques for delivering elongate shafts to a native heart valve are known in the art. For example, the elongate shaft can be delivered to the native heart valve transapically. Alternatively, and as embodied herein, the elongate shaft 401 can be delivered to the native heart valve transvascularly. For example, the elongate shaft 401 can be delivered to the native heart valve using a transseptal approach.

As embodied herein, the system 400 can include a guidewire lumen 413 extending along at least a length of the elongate shaft. As embodied herein, the elongate shaft can have a guidewire lumen 413 defined therein. Alternatively a guidewire lumen can extend along an outer wall of the elongate shaft. A guidewire can be used in combination with known surgical techniques to deliver the distal portion 403 of the system 400 to the native heart valve. For purpose of example, and as embodied herein, a guidewire can be transeptally delivered to the native mitral valve, and the distal portion 403 of the system 400 can be delivered to the native heart valve over the guidewire. Various guidewire configurations are known in the art and suitable for use with the disclosed subject matter. For example, both over-the-wire and monorail configurations can be used. For purpose of example and not limitation, the guidewire lumen can extend along at least 20 cm from the distal tip along the length of the elongate shaft 401.

The elongate shaft 401 can define one or more lumens, as described above. The elongate shaft can have any suitable construction. The materials and method of construction for the elongate shaft 401 can be selected to provide desirable performance properties of the elongate shaft 401. As described further herein, the configuration of the elongate shaft 401 can facilitate transmission of torque applied at the proximal end portion 402 of the elongate shaft to the distal end portion 403 of the elongate shaft. Further, the tensile or compressive properties of the elongate shaft 401 can impact the force transmission from the proximal end 402 to the distal end 403. The elongate shaft can have a uniform construction, or a composite construction. A composite construction, including for example, using different materials and/or constructions along the length of the elongate shaft 401 can provide desired performance characteristics (e.g., bending or torque transmission) at desired sections along the length of the elongate shaft 401. For purpose of example and not limitation, the elongate shaft construction can include polymer extrusions or assemblies of laminations such as Pebax. Additionally or alternatively, the elongate shaft construction can incorporate lumen liners such as PTFE or Polyimide.

For purpose of example, and as embodied herein, the elongate shaft 401 can include a braided reinforcement 404. The braided reinforcement 404 can be included along a portion of the elongate shaft 401, or can extend along substantially the length of the elongate shaft 401. For purpose of example and not limitation, the braided reinforcement 404 can be included between laminations. The braided reinforcement 404 can provide increased torque transmission and force transmission between the proximal end 402 and distal end 403, without compromising the integrity of the elongate shaft 401.

Additionally or alternatively, the elongate shaft can comprise a hypotube. The hypotube can include cut out portions along its length. For example, cut outs can be included to provide the hypotube with flexibility and the desired torque transmission properties. The elongate shaft can include one or more materials, including stainless steel, nitinol, or other materials known in the art for surgical applications. Examples of additional elongate shaft configurations are described in U.S. patents 4,646,719, 4,998,917, and 6,540,719.

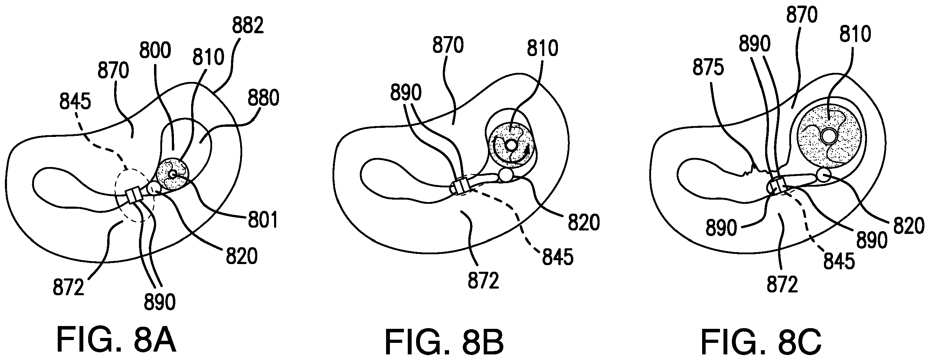

In accordance with the disclosed subject matter, a balloon 410 is disposed at the distal end portion 403 of the elongate shaft 401. The balloon 410 is inflatable from a collapsed condition to an inflated condition. For purpose of example and as embodied herein, the elongate shaft can include an outer lumen for admitting fluid and withdrawing fluid from the balloon to inflate and collapse the balloon. The elongate shaft 401 can also include a guidewire lumen 413, as described above. One or more of the outer lumen and the guidewire lumen 413 can include a braided reinforcement as described above. As embodied herein, inflation of the balloon 410 can be used to separate the native heart valve leaflets attached together by a fixation device. For example, and as described further herein with reference to FIGs.8A-8C, the balloon can have an inflation diameter in the inflated condition, the inflated balloon inflation diameter being larger than a maximum cross dimension of an orifice defined between the native heart valve leaflets to be separated.

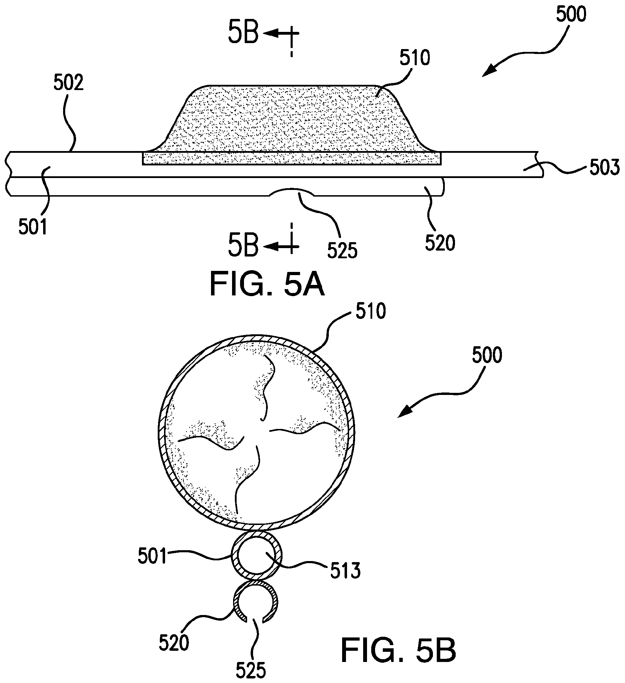

FIG. 4A depicts a balloon 410 disposed at the distal end portion 403 of the elongate shaft 401 with the balloon 410 in a collapsed condition. The system 400 can be delivered to the native heart valve with the balloon 410 in the collapsed condition. For example, the smaller diameter of the balloon in the collapsed condition can facilitate transvascular delivery of the system to, and positioning within, the native heart valve. FIG. 4B depicts the balloon 410 in an inflated condition. With reference to FIG. 4C, the balloon 410 can be concentric with the longitudinal axis of the elongate shaft 401. Alternatively, and with reference to the exemplary system 500 depicted in FIGs. 5A and 5B, the balloon 510 can be acentric with the longitudinal axis of the elongate shaft 501. As described further herein, an acentric balloon configuration can be used to control the application of forces to separate the native heart valve leaflets attached together by a fixation device.

In accordance with the disclosed subject matter, at least one snare lumen 420 extends along at least a portion of the balloon 410. As embodied herein, the at least one snare lumen 420 can extend along an exterior of the balloon 410. For purpose of example, at least one snare lumen 420 can be attached to at least one of the elongate shaft 401 and the balloon 410, and the at least one snare lumen 420 can be moveable relative to the exterior of the balloon 410 to accommodate an increased circumference of the balloon 410 as the balloon 410 inflates from the collapsed condition to the inflated condition. As embodied herein, a portion of the at least one snare lumen 420 can be attached to a distal portion of the balloon 410 with a remaining portion of the at least one snare lumen 420 moveable relative to the exterior of the balloon 410 to accommodate changing circumference of the balloon 410 and prevent restriction of the balloon as it expands. For purpose of example, the at least one snare lumen 420 can be bonded to a portion of the balloon 410. For purpose of example, the remaining portion of the at least one snare lumen 420 which remains moveable relative to the exterior of the balloon can be disposed within another lumen, which can maintain the radial position of the at least one snare lumen 420 relative to the balloon 410 while allowing the at least one snare lumen 420 to move longitudinally as the balloon expands.

In accordance with another aspect of the disclosed subject matter, the at least one snare lumen can be defined within a wall of the balloon. For example, polyimide tubing can be used to reinforce the at least one snare lumen defined within a wall of the balloon.

The configuration of the at least one snare lumen 420 can be selected based on the desired properties of the system. For example, and as described above, the construction of the at least one snare lumen 420 can be selected based on the desired torque transmission properties of the system. The at least one snare lumen 420 can have a uniform construction, or a composite construction. A composite construction, including for example, using different materials and/or constructions along the length of the at least one snare lumen 420 can provide desired performance characteristics (e.g., bending or torque transmission) at desired sections along the length of at least one snare lumen 420. For purpose of example and not limitation, the snare lumen construction can include polymer extrusions or assemblies of laminations such as Pebax. Additionally or alternatively, the at least one snare lumen 420 can be configured to increase in length axially as the balloon 410 inflates from the collapsed condition to the inflated condition to accommodate an increased circumference of the balloon 410. For example, the at least one snare lumen 420 can include elastic materials to facilitate changes in axial length of the at least one snare lumen 420 during inflation of the balloon 410. Additionally or alternatively, the at least one snare lumen 420 can include a telescoping tube construction.

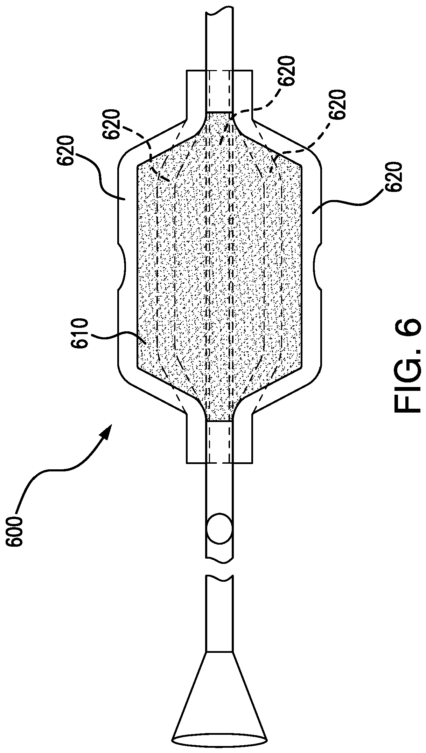

Systems in accordance with the disclosed subject matter can include any desirable number of snare lumens. For example, the at least one snare lumen can include between one and six snare lumens spaced about an outer circumference of the balloon. With reference to the exemplary system depicted in FIG. 6, the system 600 can include five snare lumens 620 spaced about an outer perimeter of balloon 610.

In accordance with the disclosed subject matter, the at least one snare lumen 420 has an exit port 425 defined therein. As embodied herein, the exit port 425 can be disposed at an intermediate location along the length of the balloon 410. For example, the balloon 410 can have an inflation diameter as described above, and a maximum inflation diameter of the balloon 410 can be at the intermediate location along the length of the balloon. The balloon 410 can apply a greater tension to the snare when the exit port 425 is disposed at an intermediate location along the length of the balloon proximate the maximum inflation diameter of the balloon 410, as described further herein. Additionally, the deployed snare can be more stable with respect to the fixation device when the snare lumen is located at a portion of the balloon 410 having the maximum inflation diameter.

As embodied herein, the system 400 can include a radiopaque marker 430 proximate the exit port. The radiopaque marker 430 can facilitate visualization and positioning of the system 400 during surgical procedures. For example, and as further described herein, the radiopaque marker 430 can facilitate orientation of the exit port 425 within the native heart valve using surgical visualization techniques known in the art.

As embodied herein, the system 400 can be configured with the balloon 410 rotatable about the longitudinal axis of the elongate shaft 401 to move the exit port 425 of the at least one snare lumen 420 circumferentially about the longitudinal axis. For example, and as described above, the elongate shaft 401 can include braided reinforcement 404 or a hypotube to facilitate transmission of torque applied at the proximal end portion 402 of the elongate shaft 401 to the distal end portion 403 of the elongate shaft 401. Accordingly, rotation of the proximal end portion 402 of the elongate shaft 401 can cause corresponding rotation of the balloon 410 at the distal end portion 403 of the elongate shaft 401. Additionally or alternatively, and in accordance with another aspect of the disclosed subject matter, the balloon 410 can be wrapped around the elongate shaft 401 in the collapsed condition and configured to unfold upon inflation from the collapsed condition to the inflated condition. Unfolding of the balloon 410 can cause rotation of the balloon 410 about the longitudinal axis of the elongate shaft 401 and corresponding movement of the exit port 425 circumferentially about the longitudinal axis. As described further herein, controlling rotation of the balloon 410 and movement of the exit port 425 circumferentially about the longitudinal axis can be desirable for separating native heart valve leaflets. For example, rotation of the balloon 410 and corresponding movement of the exit port 425 can be used to control the application of forces to separate the native heart valve leaflets As embodied herein, rotation of the balloon 410 and corresponding movement of the exit port 425 can concentrate the forces generated by inflation of the balloon 410 on the anterior leaflet tissue to tear the native mitral valve anterior leaflet.

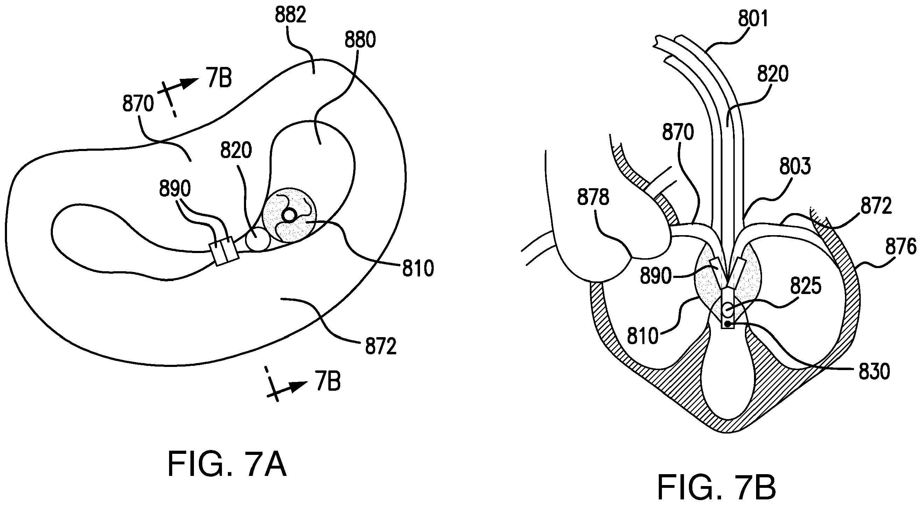

With reference to FIGS. 7B and 8A-8C, and in accordance with the disclosed subject matter, a snare 845 is deployable through the exit port 825 from a delivery position within the at least one snare lumen 820 to an extended position extending beyond the exit port 825. The snare 845 in the extended position is configured to capture the fixation devices 890. With reference to FIG. 8A, the snare 845 is depicted in an extended position extending beyond the exit port of the at least one snare lumen 820 to encircle the fixation devices 890. As embodied herein, the snare 845 can be a loop of wire. The snare 845 can be made of any suitable material. For purpose of example and as embodied herein, the snare 845 can be made from stainless steel wire, mp35, or nitinol. The snare 845 can include a continuous wire having, for example, a monofilament or braided construction. Additionally or alternatively, the snare 845 can include a closed coil exterior and central wire looped at the distal end, which can prevent elongation. The snare 845 can include a loop defined along a portion of the snare 845, for example at the snare distal end. Additionally or alternatively, the snare 845 can include a loop with both ends terminating at the proximal end. As embodied herein, the at least one snare lumen 820 and snare 845 can extend along the longitudinal axis of the elongate shaft 801 towards the proximal end portion 402 of the elongate shaft 401, and deployment and retraction of the snare 845 can be controlled from the proximal end portion 402 of the elongate shaft 401. For example, manipulation of the snare 845 from the proximal end portion 402 can result in the distal portion of the snare 845 responding in kind.

The disclosed subject matter also includes methods for separating native heart valve leaflets attached together by a fixation device. Methods in accordance with the disclosed subject matter include delivering a distal end portion of a system for separating native heart valve leaflets to a native heart valve. The system for separating native heart valve leaflets can include the features described herein. With reference to the exemplary system 800 depicted in FIG. 7A-FIG. 8C, delivering the distal end portion 803 can include positioning the balloon 810 through an orifice 880 defined between the native heart valve leaflets 870, 872. As embodied herein, the native heart valve can be a native mitral valve and the distal end portion 803 and balloon 810 of the system 800 can be positioned through a lateral orifice 880 of the native mitral valve defined between the attached native leaflets 870, 872. With reference to FIGs. 8A-8C, the lateral orifice 880 is disposed opposite the septal wall 882. As described above, the native leaflets can be attached by a previously implanted fixation device, such as a MitraClip^{®} fixation device, sold by Abbott Vascular, Santa Clara, Calif., USA. Various surgical access routes can be used to access the native heart valve in accordance with the disclosed subject matter. For example, the distal end portion 803 can be delivered to the native heart valve transapically, or as embodied herein, the distal end portion 803 can be delivered transseptally. The system 800 can include a steerable catheter for transseptal delivery to and positioning within the native mitral valve.

Methods for separating native heart valve leaflets attached together by a fixation device in accordance with the disclosed subject matter further include deploying a snare 845 from a delivery position within the at least one snare lumen to an extended position extending beyond the exit port 825. As embodied herein, deploying the snare can include rotating the balloon 810 about the longitudinal axis of the elongate shaft 801 to align the exit port 825 towards the fixation devices 890. For example, and with reference to FIG. 7B, the exemplary system 800 is depicted with the exit port 825 aligned towards the fixation devices 890. As discussed above, the system can include one or more markers, such as a radiopaque marker 830, which can be used to position the system for deployment of the snare using known visualization techniques.

Methods in accordance with the disclosed subject matter further include capturing the fixation devices 890 within the snare 845 with the snare 845 in the extended position. As described above, the snare 845 and snare lumen 820 can extend along the longitudinal axis of the elongate shaft 801, and deployment and retraction of the snare 845 can be controlled from the proximal end portion 802 of the elongate shaft 801. Additionally, and as further embodied herein, the balloon 810 and snare lumen 820 can be vertically and rotationally aligned within the native heart valve to deploy the snare 845 and capture the fixation devices 890 with the snare 845 in the extended position. For example, the system 800 can be positioned vertically within the native heart valve prior to snare deployment. For example, and as embodied herein, the system can be positioned with the exit port 825 underneath the native heart valve leaflets 870, 872 and vertically aligned beneath a lower portion of the fixation devices 890 when the heart is viewed in cross section. As embodied herein, the snare 845 can be deployed with the exit port 825 rotationally aligned towards the fixation devices 890 and vertically aligned below a lower portion of the fixation devices 890. With the snare 845 deployed, the system 800 can be retracted along the longitudinal axis of the elongate shaft 801 to adjust the vertical position of the system 800 within the native heart valve and place the snare 845 around the fixation devices 890. With reference to FIG. 8A, the exemplary system 800 is depicted with the snare 845 deployed in the extended position extending beyond the exit port 825 and encircling the fixation devices 890 to capture the fixation devices 890 within the snare 845. FIG. 8A is a taken from the atrial side of the mitral valve, and therefore, the snare 845 is depicted in broken line where the snare extends beneath the native leaflets 870, 872 for purpose of explanation. As embodied herein, the snare 845 can be comprised of material that is visible using surgical imaging techniques known in the art, and visualization techniques can be used during deployment of the snare 845 to facilitate capture of the fixation devices 890. As embodied herein, the snare 845 can be deployed and positioned around the fixation devices 890 prior to inflation of the balloon 810.

With reference to FIGs. 8B & 8C, methods in accordance with the disclosed subject matter include inflating the balloon 810 from a collapsed condition to an inflated condition to separate the native heart valve leaflets 870, 872. As embodied herein, the balloon 810 can be inflated after the fixation devices 890 are captured within the snare 845. As described above, the balloon 810 can have an inflation diameter in the inflated condition, the balloon inflation diameter being larger than a maximum cross dimension of an orifice 880 defined between the native heart valve leaflets 870, 872 to be separated. As embodied herein, the method can include rotating the balloon 810 about the longitudinal axis of the elongate shaft 801 to align the exit port 825 away from the native mitral valve anterior leaflet 870 after capturing the fixation devices 890 in the snare 845. As described above, aligning the exit port 825 away from the anterior leaflet 870 after capturing the fixation devices 890 with the snare 845 can cause the balloon 810 to apply tension to the snare 845 and fixation devices 890 captured therein in a direction extending away from the anterior leaflet 870 as the balloon 810 inflates. Applying tension to the fixation devices 890 in a direction extending away from the anterior leaflet 870 during balloon inflation can separate the native valve leaflets by tearing the anterior mitral valve leaflet 870. With reference to FIG. 8C, the system 800 is depicted with the balloon 810 in the inflated condition and the native mitral valve leaflets separated by a tear 875 through the native mitral valve anterior leaflet tissue 870.

The balloon 810 can be rotated prior to or during inflation of the balloon from the collapsed condition to the inflated condition. For example, and as described above, the balloon 810 can be configured to unfold as the balloon 810 inflates, and the unfolding can cause rotation of the balloon and corresponding rotation of the at least one snare lumen 820 and exit port 825 to align the exit port 825 away from the native mitral valve anterior leaflet 870. Additionally or alternatively, the elongate shaft 801 can include a braided reinforcement and/or additional features to transmit torque from the proximal portion 802 of the elongate shaft 801 to the balloon 810.

In accordance with another aspect of the disclosed subject matter, and as described above with reference to FIGs. 5A and 5B, the balloon 510 can be acentric with the longitudinal axis of the elongate shaft 501. The exemplary system 500 includes an elongate shaft 501 having a proximal end portion 502, a distal end portion 503 and a longitudinal axis extending therebetween. The elongate shaft 501 is configured for transvascular delivery of the distal end portion 503 to a native heart valve. A balloon 510 is disposed at the distal end portion 503 of the elongate shaft 501 , and the balloon 510 is inflatable from a collapsed condition to an inflated condition. At least one snare lumen 520 extends along the longitudinal axis along at least a portion of the balloon 510, the at least one snare lumen 520 having an exit port 525 defined therein. A snare is deployable through the exit port 525 from a delivery position within the at least one snare lumen 520 to an extended position extending beyond the exit port 525. The snare in the extended position is configured to capture the fixation device. As described above, the system 500 can include a guidewire lumen 513 extending along at least a length of the elongate shaft. As embodied herein, the balloon 510 can be configured to inflate away from the at least one snare lumen 520 and the exit port 525. An acentric balloon configuration can concentrate forces generated by inflation of the balloon 510 on the anterior leaflet tissue to tear the anterior leaflet during inflation of the balloon 510. As embodied herein, the exit port 525 can be aligned away from the anterior leaflet after capturing the fixation device in the snare, and inflation of the acentric balloon 510 can apply tension to the snare in a direction extending away from the anterior leaflet to tear the anterior leaflet during balloon inflation.

As described above, separation of native heart valve leaflets attached by a fixation device can be desirable for a number of clinical reasons. For example, sometimes after a fixation device is installed mitral valve regurgitation can still exist, or can arise again. In some cases, it can be desirable to remove the fixation device to allow for implantation of a replacement heart valve. Accordingly, and in accordance with another aspect of the disclosed subject matter, after separating the native heart valve leaflets as described herein, a prosthetic heart valve can be installed at the native heart valve. The prosthetic heart valve can be delivered to and installed at the native heart valve using techniques known in the art. For example, the prosthetic heart valve can be delivered transapically, or alternatively, can be delivered transvascularly, including transeptally. Examples of prosthetic heart valves and methods for delivering prosthetic heart valves are described in U.S. patents 10,470,881, 9,439,757, and 8,870,948.

As embodied herein, the fixation devices 890 can remain attached to the native mitral valve posterior leaflet 872 after the native heart leaflets 870, 872 are separated. Having the fixation devices 890 remain attached to the posterior leaflet 872 can be desirable, including for reducing the risk of left ventricular outflow tract obstruction ("LVOTO"). For example, installation of a prosthetic valve at the native mitral valve after separation of the native heart leaflets 870, 872 can trap the fixation devices 890 and native posterior leaflet 872 against the ventricular wall 876 away from the aortic valve 878 and left ventricular outflow tract, which can reduce the risk of LVOTO.

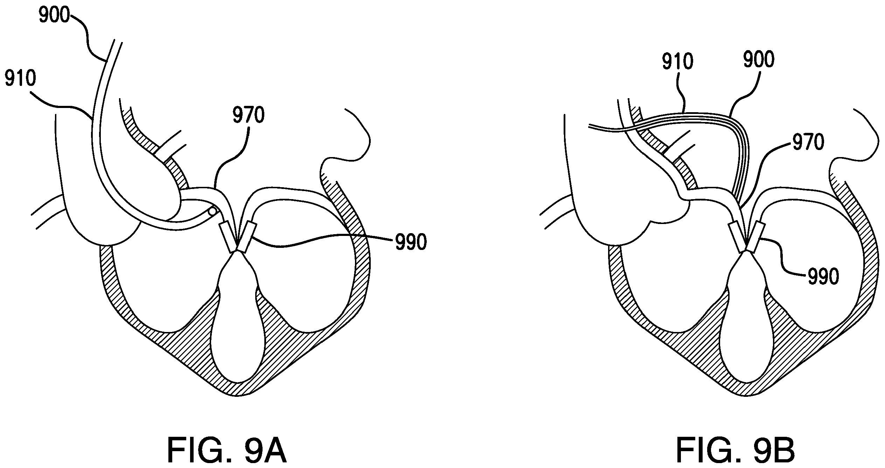

Methods in accordance with the disclosed subject matter can include additional features. For example, methods can include weakening native mitral valve anterior leaflet tissue before inflating the balloon. As described above, it can be desirable to separate the native heart valve leaflets by tearing the native mitral valve anterior leaflet tissue. Weakening the native mitral valve anterior leaflet tissue can help ensure that the native mitral valve anterior leaflet tissue tears when the balloon is inflated. Any suitable means for weakening the native anterior leaflet tissue can be used. For example, one or more of a needle, blade, and hydrogen peroxide can be applied to the native mitral valve anterior leaflet tissue to weaken the tissue prior to balloon inflation. As embodied herein, RF energy can be used to weaken the native mitral valve anterior leaflet tissue adjacent to the fixation device. Weakening the anterior leaflet tissue adjacent to the fixation device can reduce the amount of anterior leaflet tissue that remains captured in the fixation device after the native leaflets are separated. With reference to FIGs. 9A and 9B, a device 900 can be delivered to the native mitral valve anterior leaflet 970 coapted to the posterior leaflet by a fixation device 990, as described above. The device 900 can be used to weaken the tissue of the anterior leaflet 970. As embodied herein, the device 900 can be separate from the system for separating the native heart valve leaflets, and can be delivered to the native mitral valve anterior leaflet 970 transvascularly, using a transseptal approach or an aortic approach. As embodied herein, the device 900 can include a steerable catheter 910 to deliver and position the device 900. In accordance with another aspect of the disclosed subject matter, the system for separating the native heart valve leaflets can include one or more of the features described herein for weakening the native mitral valve anterior leaflet prior to balloon inflation. For example, the system can include a lumen for applying hydrogen peroxide to the native anterior leaflet 970.

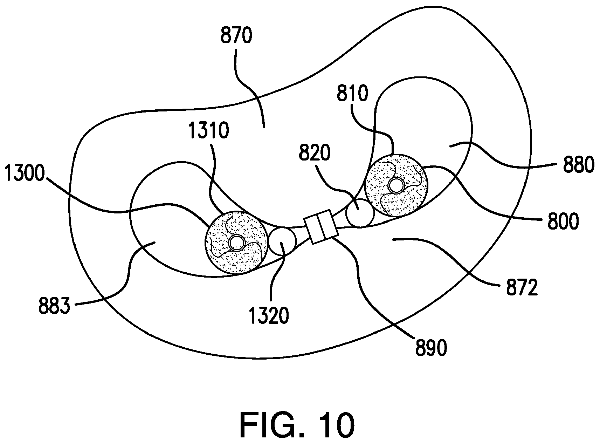

In accordance with another aspect of the disclosed subject matter, a second balloon can be positioned through a second orifice on an opposing side of the fixation device. For purpose of example and as embodied herein, the first and second balloons can each be part of a system for separating native heart valve leaflets as described herein. With reference to FIG. 10, system 800 having a balloon 810 and a snare lumen 820 can be positioned through lateral orifice 880, and system 1300 having a balloon 1310 and a snare lumen 1320 can be positioned through medial orifice 883 on an opposing side of the fixation devices 890. The first balloon 810 and the second balloon 1310 can be inflated to separate the native heart valve leaflets 870, 872.

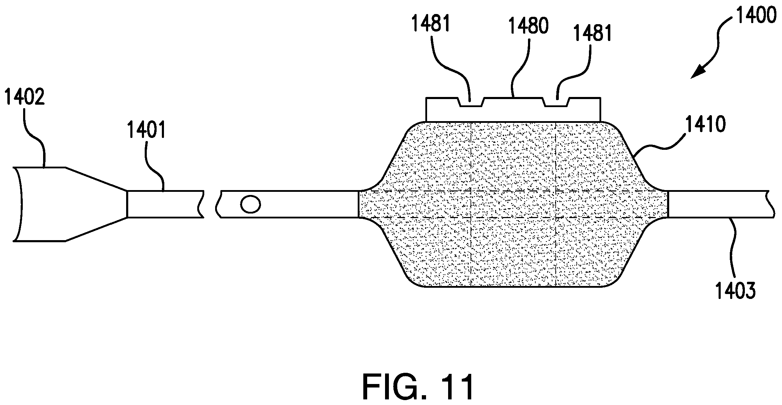

Additionally or alternatively, and in accordance with another aspect of the disclosed subject matter, the system for separating native heart valve leaflets can include at least one cutter. With reference to FIG. 11, the system 1400 includes an elongate shaft 1401 having a proximal end portion 1402, a distal end portion 1403 and a longitudinal axis extending therebetween. The elongate shaft 1401 is configured for transvascular delivery of the distal end portion 1403 to a native heart valve. A balloon 1410 is disposed at the distal end portion 1403 of the elongate shaft 1401, and the balloon 1410 is inflatable from a collapsed condition to an inflated condition. A cutter 1480 is disposed along at least a portion of the balloon 1410. As embodied herein, the cutter 1480 can extend along a length of the balloon 1410 generally parallel to the longitudinal axis of the elongate shaft 1410. In accordance with another aspect of the disclosed subject matter, the cutter can extend along the balloon in a spiral. Additionally, and as embodied herein, the cutter 1480 can include one or more serrations 1481. The cutter 1410 can be used to cut or tear native heart valve leaflet tissue when the balloon 1410 is inflated to the inflated condition to separate native heart valve leaflets. For example, inflation of the balloon can force the cutter 1481 into contact with native heart valve leaflet tissue. As embodied herein, the at least one cutter 1481 can be aligned with the native mitral valve anterior leaflet prior to inflation of the balloon 1410 at the native mitral valve. Aligning the cutter 1481 with the native mitral valve anterior leaflet can cause the cutter 1481 to cut or tear the mitral valve anterior leaflet when the balloon 1410 is inflated.

As described above, the system 1410 can include at least one snare lumen extending along at least a portion of the balloon, the at least one snare lumen having an exit port defined therein. The cutter 1480 can be disposed between about 30 degrees and 180 degrees about an outer circumference of the balloon from the at least one snare lumen. For purpose of example and not limitation, the at least one snare lumen can be positioned at approximately the 9 o'clock position when the system 1410 is viewed in end view, and the cutter 1480 can be positioned at approximately the 10 o'clock or 11 o'clock position. Positioning the cutter 1480 between about 30 degrees and 180 degrees about an outer circumference of the balloon from the at least one snare lumen can align the cutter with the native mitral valve anterior leaflet once the system 1400 has been rotated about the longitudinal axis of the elongate shaft to align the exit port away from the native mitral valve anterior leaflet after capturing the fixation device.

The disclosed subject matter further includes a kit for treating a native heart valve having leaflets attached together by a fixation device. The kit includes a system for separating native heart valve leaflets attached together by a fixation device. The system can include the features described above. The kit also includes a prosthetic heart valve.

The disclosure also includes the following examples, which are useful for understanding the invention:
In one example, there is a system for separating native heart valve leaflets attached together by a fixation device, comprising: an elongate shaft having a proximal end portion, a distal end portion and a longitudinal axis extending therebetween, the elongate shaft configured for transvascular delivery of the distal end portion to a native heart valve; a balloon disposed at the distal end portion of the elongate shaft, the balloon inflatable from a collapsed condition to an inflated condition; at least one snare lumen extending along at least a portion of the balloon, the at least one snare lumen having an exit port defined therein; and a snare deployable through the exit port from a delivery position within the at least one snare lumen to an extended position extending beyond the exit port, the snare in the extended position configured to capture the fixation device.

The elongate shaft may be torquable about the longitudinal axis to rotate the distal end portion of the elongate shaft by rotation of the proximal end portion to move the exit port of the at least one snare lumen circumferentially about the longitudinal axis.

In another example, there is a method for separating native heart valve leaflets attached together by a fixation device, the method comprising: delivering a distal end portion of a system for separating native heart valve leaflets to a native heart valve, the system including: an elongate shaft having a proximal end portion, the distal end portion, and a longitudinal axis extending therebetween; a balloon disposed at the distal end portion; and at least one snare lumen extending along at least a portion of the balloon, the at least one snare lumen having an exit port defined therein; deploying a snare from a delivery position within the at least one snare lumen to an extended position extending beyond the exit port; capturing the fixation device within the snare with the snare in the extended position; and inflating the balloon from a collapsed condition to an inflated condition to separate the native heart valve leaflets.

Delivering the distal end portion may include positioning the balloon through an orifice defined between the native heart valve leaflets.

The method may further comprise positioning a second balloon through a second orifice on an opposing side of the fixation device, and inflating the second balloon.

The native heart valve may be a mitral valve.

Delivering the distal end portion may include positioning the balloon through a lateral orifice defined between the native mitral valve leaflets.

The balloon may include a cutter disposed along at least a portion thereof, the method comprising aligning the least one cutter with the native mitral valve anterior leaflet.

Deploying the snare may include rotating the balloon about the longitudinal axis of the elongate shaft to align the exit port towards the fixation device.

The method may further comprise rotating the balloon about the longitudinal axis of the elongate shaft to align the exit port away from the native mitral valve anterior leaflet after capturing the fixation device.

Inflating the balloon may tear the native mitral valve anterior leaflet.

The fixation device may remain attached to the native mitral valve posterior leaflet.

The distal end may be delivered transapically.

The distal end may be delivered transseptally.

The method may further comprise weakening native mitral valve anterior leaflet tissue before inflating the balloon.

At least one of a needle, blade, RF energy, and hydrogen peroxide may be applied to the native mitral valve anterior leaflet tissue in order to weaken it.

The method may further comprise installing a prosthetic heart valve at the native heart valve.

In addition to the specific embodiments claimed below, the disclosed subject matter is also directed to other embodiments of the dependent features claimed below and those disclosed above. As such, the particular features presented in the dependent claims and disclosed above can be combined with each other in other manners within the scope of the disclosed subject matter such that the disclosed subject matter should be recognized as also specifically directed to other embodiments. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the method and system of the disclosed subject matter.

## Claims

1. A system (400, 500, 600, 1400) for separating native heart valve leaflets (870, 872) attached together by a fixation device (301, 890, 990), comprising:
an elongate shaft (401, 501, 801, 1401) having a proximal end portion (402, 502, 802, 1402), a distal end portion (403, 503, 803, 1403) and a longitudinal axis extending therebetween, the elongate shaft configured for transvascular delivery of the distal end portion to a native heart valve;
a balloon (410, 510, 610, 810, 1310, 1410) disposed at the distal end portion of the elongate shaft, the balloon inflatable from a collapsed condition to an inflated condition;
at least one snare lumen (420, 520, 620, 820, 1320) extending along at least a portion of the balloon, the at least one snare lumen having an exit port (425, 525, 825) defined therein, wherein the exit port is disposed at an intermediate location along a length of the balloon; and
a snare (845) deployable through the exit port from a delivery position within the at least one snare lumen to an extended position extending beyond the exit port, the snare in the extended position configured to capture the fixation device.

2. The system of claim 1, wherein the elongate shaft comprises one or more of i) a braided reinforcement (404); ii) a hypotube; and iii) at least one of stainless steel, nitinol, mp35, polyimide, pebax, and braid reinforced polyimide, and optionally, wherein the system further comprises a guidewire lumen (413, 513) extending along at least a length of the elongate shaft.

3. The system of any one of claims 1-2, wherein the elongate shaft is configured to transmit torque along the longitudinal axis and wherein the exit port of the at least one snare lumen can be moved circumferentially about the longitudinal axis by rotation of the proximal end portion.

4. The system of any one of claims 1-3, wherein the at least one snare lumen extends along an exterior of the balloon and is bonded to a portion of the balloon, and optionally, wherein the balloon is rotatable about the longitudinal axis of the elongate shaft to move the exit port of the at least one snare lumen circumferentially about the longitudinal axis.

5. The system of any one of claims 1-4, wherein a segment of the at least one snare lumen is attached to at least one of the elongate shaft and the balloon, and the at least one snare lumen extends along an exterior of the balloon and is movable relative the exterior of the balloon as the balloon inflates from the collapsed condition to the inflated condition to accommodate an increased circumference of the balloon.

6. The system of any one of claims 1-5, wherein the at least one snare lumen is defined within a wall of the balloon.

7. The system of any one of claims 1-6, wherein the at least one snare lumen is configured to increase in length axially as the balloon inflates from the collapsed condition to the inflated condition to accommodate an increased circumference of the balloon.

8. The system of an one of claims 1-7, wherein at least one of the balloon and the at least one snare lumen includes a radiopaque marker (430, 830) proximate the exit port, and optionally, wherein the at least one snare lumen includes between one and four snare lumens spaced about an outer circumference of the balloon.

9. The system of any one of claims 1-8, wherein the balloon is concentric with the longitudinal axis of the elongate shaft.

10. The system of any one of claims 1-9, wherein the balloon has an inflation diameter in the inflated condition; the inflation diameter being larger than a maximum cross dimension of an orifice (880) defined between the native heart valve leaflets (870, 872) to be separated, and optionally, wherein the balloon is configured to unfold upon inflation from the collapsed condition to the inflated condition.

11. The system of any one of claims 1-8 and 10, wherein the balloon is acentric with the longitudinal axis of the elongate shaft.

12. The system of any one of claims 1-11, further comprising at least one cutter (1480) disposed along at least a portion of the balloon.

13. The system of claim 12, wherein the cutter is disposed between about 30 degrees and 180 degrees about an outer circumference of the balloon from the at least one snare lumen.

14. A kit for treating a native heart valve having leaflets (870, 872) attached together by a fixation device (301, 890, 990), the kit comprising:
a system (400, 500, 600, 1400) for separating native heart valve leaflets attached together by a fixation device according to any one of claims 1-13; and
a prosthetic heart valve.

## Patentansprüche

1. System (400, 500, 600, 1400) zum Trennen von durch eine Fixierungsvorrichtung (301, 890, 990) miteinander verbundenen nativen Herzklappensegeln (870, 872), umfassend:
einen langgestreckten Schaft (401, 501, 801, 1401) mit einem proximalen Endabschnitt (402, 502, 802, 1402), einem distalen Endabschnitt (403, 503, 803, 1403) und einer sich dazwischen erstreckenden Längsachse, wobei der langgestreckte Schaft zur transvaskulären Zuführung des distalen Endabschnitts zu einer nativen Herzklappe konfiguriert ist;
einen Ballon (410, 510, 610, 810, 1310, 1410), der an dem distalen Endabschnitt des langgestreckten Schafts angeordnet ist, wobei der Ballon von einem kollabierten Zustand in einen aufgeblasenen Zustand aufblasbar ist;
mindestens ein Schlingenlumen (420, 520, 620, 820, 1320), das sich entlang mindestens eines Teils des Ballons erstreckt, wobei das mindestens eine Schlingenlumen eine darin definierte Ausgangsöffnung (425, 525, 825) aufweist, wobei die Ausgangsöffnung an einer intermediären Stelle entlang einer Länge des Ballons angeordnet ist; und
eine Schlinge (845), die durch die Ausgangsöffnung aus einer Abgabeposition innerhalb des mindestens einen Schlingenlumens in eine ausgefahrene Position entfaltbar ist, die sich über die Ausgangsöffnung hinaus erstreckt, wobei die Schlinge in der ausgefahrenen Position konfiguriert ist, um die Fixierungsvorrichtung einzufangen.

2. System nach Anspruch 1, bei dem der langgestreckte Schaft eines oder mehrere von i) einer geflochtenen Verstärkung (404); ii) einem Hypotube; und iii) mindestens einem von rostfreiem Stahl, Nitinol, mp35, Polyimid, Pebax und geflochtenem verstärktem Polyimid umfasst, und optional, bei dem das System ferner ein Führungsdrahtlumen (413, 513) umfasst, das sich entlang mindestens einer Länge des langgestreckten Schafts erstreckt.

3. System nach einem der Ansprüche 1 - 2, bei dem der langgestreckte Schaft konfiguriert ist, um ein Drehmoment entlang der Längsachse zu übertragen, und bei dem die Ausgangsöffnung des mindestens einen Schlingenlumens durch Drehung des proximalen Endabschnitts in Umfangsrichtung um die Längsachse bewegbar ist.

4. System nach einem der Ansprüche 1 - 3, bei dem sich das mindestens eine Schlingenlumen entlang einer Außenseite des Ballons erstreckt und mit einem Teil des Ballons verbunden ist, und optional, bei dem der Ballon um die Längsachse des langgestreckten Schafts drehbar ist, um die Austrittsöffnung des mindestens einen Schlingenlumens in Umfangsrichtung um die Längsachse zu bewegen.

5. System nach einem der Ansprüche 1 - 4, bei dem ein Segment des mindestens einen Schlingenlumens an dem langgestreckten Schaft und/oder dem Ballon befestigt ist und das mindestens eine Schlingenlumen sich entlang einer Außenseite des Ballons erstreckt und relativ zur Außenseite des Ballons beweglich ist, wenn sich der Ballon vom kollabierten Zustand in den aufgeblasenen Zustand ausdehnt, um einen vergrößerten Umfang des Ballons zu ermöglichen.

6. System nach einem der Ansprüche 1 - 5, bei dem das mindestens eine Schlingenlumen innerhalb einer Wand des Ballons definiert ist.

7. System nach einem der Ansprüche 1 - 6, bei dem das mindestens eine Schlingenlumen konfiguriert ist, um axial an Länge zuzunehmen, wenn sich der Ballon vom kollabierten Zustand in den aufgeblasenen Zustand ausdehnt, um einen vergrößerten Umfang des Ballons unterzubringen.

8. System nach einem der Ansprüche 1 - 7, bei dem der Ballon und/oder das mindestens eine Schlingenlumen eine röntgendichte Markierung (430, 830) in der Nähe der Ausgangsöffnung aufweist, und optional, bei dem das mindestens eine Schlingenlumen zwischen einem und vier Schlingenlumen umfasst, die um den Außenumfang des Ballons herum beabstandet sind.

9. System nach einem der Ansprüche 1 - 8, bei dem der Ballon konzentrisch mit der Längsachse des langgestreckten Schafts ist.

10. System nach einem der Ansprüche 1 - 9, bei dem der Ballon im aufgeblasenen Zustand einen Aufblasdurchmesser aufweist, der größer ist als die maximale Querabmessung einer Öffnung (880), die zwischen den zu trennenden nativen Herzklappensegeln (870, 872) definiert ist, und bei dem der Ballon optional konfiguriert ist, um sich beim Aufblasen vom kollabierten Zustand in den aufgeblasenen Zustand zu entfalten.

11. System nach einem der Ansprüche 1 - 8 und 10, bei dem der Ballon azentrisch zur Längsachse des langgestreckten Schafts angeordnet ist.

12. System nach einem der Ansprüche 1 - 11, das ferner mindestens eine Schneidvorrichtung (1480) umfasst, die entlang mindestens eines Teils des Ballons angeordnet ist.

13. System nach Anspruch 12, bei dem die Schneidvorrichtung zwischen etwa 30 Grad und 180 Grad um einen Außenumfang des Ballons von dem mindestens einen Schlingenlumen angeordnet ist.

14. Teilesatz zur Behandlung einer nativen Herzklappe mit durch eine Fixierungsvorrichtung (301, 890, 990) aneinander befestigten Segeln (870, 872), wobei der Teilesatz umfasst:
ein System (400, 500, 600, 1400) zum Trennen nativer Herzklappensegel, die durch eine Fixierungsvorrichtung aneinander befestigt sind, nach einem der Ansprüche 1-13; und
eine Herzklappenprothese.

## Revendications

1. Système (400, 500, 600, 1400) pour la séparation de feuillets valvulaires cardiaques natifs (870, 872) attachés ensemble par un dispositif de fixation (301, 890, 990), comprenant :
un arbre allongé (401, 501, 801, 1401) ayant une partie d'extrémité proximale (402, 502, 802, 1402), une partie d'extrémité distale (403, 503, 803, 1403) et un axe longitudinal s'étendant entre elles, l'arbre allongé étant configuré pour une administration transvasculaire de la partie d'extrémité distale à une valve cardiaque native ;
un ballonnet (410, 510, 610, 810, 1310, 1410) disposé au niveau de la partie d'extrémité distale de l'arbre allongé, le ballonnet étant gonflable d'un état plié à un état gonflé ;
au moins une lumière de piège (420, 520, 620, 820, 1320) s'étendant le long d'au moins une partie du ballonnet, l'au moins une lumière de piège ayant un orifice de sortie (425, 525, 825) défini en son sein, dans lequel l'orifice de sortie est disposé au niveau d'un emplacement intermédiaire le long d'une longueur du ballonnet ; et
un piège (845) déployable à travers l'orifice de sortie à partir d'une position d'administration à l'intérieur de l'au moins une lumière de piège vers une position étendue s'étendant au-delà de l'orifice de sortie, le piège dans la position étendue étant configuré pour capturer le dispositif de fixation.

2. Système selon la revendication 1, dans lequel l'arbre allongé comprend un ou plusieurs de i) un renforcement tressé (404) ; ii) un hypotube ; et iii) au moins l'un d'acier inoxydable, de Nitinol, mp35, polyimide, Pebax, et polyimide renforcé par une tresse, et optionnellement, dans lequel le système comprend en outre une lumière de fil de guidage (413, 513) s'étendant le long d'au moins une longueur de l'arbre allongé.

3. Système selon l'une quelconque des revendications 1 à 2, dans lequel l'arbre allongé est configuré pour transmettre un couple le long de l'axe longitudinal et dans lequel l'orifice de sortie de l'au moins une lumière de piège peut être déplacé circonférentiellement autour de l'axe longitudinal par rotation de la partie d'extrémité proximale.

4. Système selon l'une quelconque des revendications 1 à 3, dans lequel l'au moins une lumière de piège s'étend le long d'un extérieur du ballonnet et est collée à une partie du ballonnet, et optionnellement, dans lequel le ballonnet est rotatif autour de l'axe longitudinal de l'arbre allongé pour déplacer l'orifice de sortie de l'au moins une lumière de piège circonférentiellement autour de l'axe longitudinal.

5. Système selon l'une quelconque des revendications 1 à 4, dans lequel un segment de l'au moins une lumière de piège est attaché à au moins l'un de l'arbre allongé et du ballonnet, et l'au moins une lumière de piège s'étend le long d'un extérieur du ballonnet et est mobile par rapport à l'extérieur du ballonnet lorsque le ballonnet gonfle de l'état plié à l'état gonflé pour recevoir une circonférence augmentée du ballonnet.

6. Système selon l'une quelconque des revendications 1 à 5, dans lequel l'au moins une lumière de piège est définie à l'intérieur d'une paroi du ballonnet.

7. Système selon l'une quelconque des revendications 1 à 6, dans lequel l'au moins une lumière de piège est configurée pour augmenter en longueur axialement lorsque le ballonnet gonfle de l'état plié à l'état gonflé pour recevoir une circonférence augmentée du ballonnet.

8. Système selon l'une quelconque des revendications 1 à 7, dans lequel au moins un du ballonnet et de l'au moins une lumière de piège comporte un marqueur radio-opaque (430, 830) à proximité de l'orifice de sortie, et éventuellement, dans lequel l'au moins une lumière de piège comporte entre une et quatre lumières de piège espacées autour d'une circonférence externe du ballonnet.

9. Système selon l'une quelconque des revendications 1 à 8, dans lequel le ballonnet est concentrique avec l'axe longitudinal de l'arbre allongé.

10. Système selon l'une quelconque des revendications 1 à 9, dans lequel le ballonnet a un diamètre de gonflage dans l'état gonflé ; le diamètre de gonflage est supérieur à une dimension transversale maximale d'un orifice (880) défini entre les feuillets valvulaires cardiaques natifs (870, 872) devant être séparés, et optionnellement, dans lequel le ballonnet est configuré pour se déplier lors du gonflage de l'état plié à l'état gonflé.

11. Système selon l'une quelconque des revendications 1 à 8 et 10, dans lequel le ballonnet est acentrique avec l'axe longitudinal de l'arbre allongé.

12. Système selon l'une quelconque des revendications 1 à 11, comprenant en outre au moins un dispositif de coupe (1480) disposé le long d'au moins une partie du ballonnet.

13. Système selon la revendication 12, dans lequel le dispositif de coupe est disposé entre environ 30 degrés et 180 degrés autour d'une circonférence externe du ballonnet à partir de l'au moins une lumière de piège.

14. Kit de traitement d'une valve cardiaque native ayant des feuillets (870, 872) attachés ensemble par un dispositif de fixation (301, 890, 990), le kit comprenant :
un système (400, 500, 600, 1400) pour séparer des feuillets de valve cardiaque native attachés ensemble par un dispositif de fixation selon l'une quelconque des revendications 1 à 13 ; et
une valve cardiaque prothétique.
